# EUROPÄISCHE PATENTANMELDUNG

(11) **EP 3 939 488 A1**
(43) Veröffentlichungstag der Anmeldung: **19.01.2022**
(21) Anmeldenummer: 21179100.9
(22) Anmeldetag: 11.06.2021
(51) Int. Cl.: A61B 1/00

(54) **STEREOENDOSKOP**

(30) Priorität: 16.07.2020 DE 102020118814
(71) Anmelder: avateramedical GmbH, 07745 Jena (DE)
(72) Erfinder: WEISE, Fabian, 10437 Berlin (DE); BEYER, Stefan, 10969 Berlin (DE)
(74) Vertreter: Gleim Petri Oehmke Patent- und Rechtsanwaltspartnerschaft mbB

(57) **Zusammenfassung**

Die Erfindung betrifft ein Stereoendoskop zur Beobachtung und Analyse eines Objekts. Ein solches Stereoendoskop umfasst einen Schaft (3) mit einem distalen Ende (1) und einem proximalen Ende (2). Es umfasst außerdem Beleuchtungsmittel zur Beleuchtung des Objekts sowie stereoskopische Abbildungsmittel, welche vom Objekt abgestrahltes Licht vom distalen Ende (1) zum proximalen Ende (2) übertragen. Licht eines ersten Abbildungskanals (7) wird in eine erste Sensoreinheit (15) eingespeist, Licht aus einem zweiten Abbildungskanal (8) in eine zweite Sensoreinheit (16). In beiden Sensoreinheiten (15, 16) befinden sich voneinander verschiedene Strahlteiler (17, 20), welche das Licht in insgesamt vier Lichtstrahlen (41, 42, 43, 44) aufteilen. Je ein Lichtstrahl (42, 43) aus jeder der Sensoreinheiten (15, 16) wird auf je einen Sensor (19, 21) gelenkt, diese beiden Sensoren (19, 21) sind identisch. Die dort detektieren Signale werden zu einem stereoskopischen Bild zusammengesetzt.

Erfindungsgemäß umfasst das Stereoendoskop voneinander verschiedenartige Manipulationsmittel für die beiden anderen Lichtstrahlen (41, 44) und mittels entsprechend abgestimmter Bildverarbeitungsalgorithmen lassen sich zwei verschiedene monoskopische Bilder erzeugen, welche zueinander und zum stereoskopischen Bild komplementäre Bildinformationen enthalten.

## Beschreibung

### Gebiet der Erfindung

Die Erfindung betrifft ein Stereoendoskop zur Beobachtung und Analyse eines Objekts. Ein solches Stereoendoskop umfasst einen Schaft mit einem distalen Ende und einem proximalen Ende. Es umfasst außerdem Beleuchtungsmittel, welche das Objekt vom distalen Ende des Schaftes her beleuchten. Das Stereoendoskop umfasst außerdem stereoskopische Abbildungsmittel, welche vom Objekt abgestrahltes Licht vom distalen Ende zum proximalen Ende übertragen. Weitere Bestandteile des Stereoendoskops bilden eine erste und eine zweite Sensoreinheit. Auf die erste Sensoreinheit wird Licht aus einem ersten Abbildungskanal abgebildet. Sie umfasst einen ersten Strahlteiler, welcher das Licht in einen ersten Lichtstrahl und in einen zweiten Lichtstrahl aufteilt. Die erste Sensoreinheit umfasst außerdem einen ersten und einen zweiten Sensor. Der erste Sensor detektiert Licht des ersten Lichtstrahls und wandelt dieses in erste elektromagnetische Signale um. Der zweite Sensor detektiert Licht des zweiten Lichtstrahls und wandelt dieses in zweite elektromagnetische Signale um. Licht aus einem zweiten Abbildungskanal wird auf die zweite Sensoreinheit abgebildet. Die zweite Sensoreinheit umfasst einen zweiten Strahlteiler, welcher das Licht in einen dritten Lichtstrahl und einen vierten Lichtstrahl aufteilt. Die zweite Sensoreinheit umfasst außerdem einen dritten und einen vierten Sensor. Der dritte Sensor detektiert Licht des dritten Lichtstrahls und wandelt dieses in dritte elektromagnetische Signale um. Der vierte Sensor detektiert Licht des vierten Lichtstrahls und wandelt dieses in vierte elektromagnetische Signale um. Der zweite Sensor und der dritte Sensor sind gleichartig und detektieren jeweils Licht im sichtbaren Spektralbereich. Schließlich umfasst das Stereoendoskop auch eine Bildverarbeitungseinheit zur Verarbeitung der ersten bis vierten elektromagnetischen Signale. Die zweiten und dritten elektromagnetischen Signale werden von der Bildverarbeitungseinheit dabei zu einem stereoskopischen Bild verarbeitet.

### Stand der Technik

Im Stand der Technik sind eine Reihe von Stereoendoskopen bekannt, welche zum Teil auch mit verschiedenen Bildverarbeitungstechniken kombiniert werden. In der US 5,776,049 A wird ein Stereoendoskop beschrieben, bei welchem die Bilder für den linken und den rechten Kanal mittels identischer Übertragungssysteme durch den Schaft des Stereoendoskops vom distalen zum proximalen Ende übertragen werden, wo sie dann nach dem Durchlaufen entsprechender Abbildungsoptiken auf Flächendetektoren abgebildet werden.

In der US 2019/0200848 A1 wird ein Stereoendoskop als Teil eines robotischen Operationssystems beschrieben. Damit lassen sich zum einen stereoskopische Bilder im sichtbaren Bereich aufnehmen und zum anderen hyperspektrale stereoskopische Bilder. Bei der Aufnahme von hyperspektralen Bildern wird ein Wellenlängenbereich, der insbesondere auch Wellenlängen aus nicht sichtbaren Wellenlängenbereichen umfassen kann, in eine Vielzahl schmaler spektraler Kanäle aufgeteilt. Für jeden spektralen Kanal wird ein eigenes Bild aufgenommen, so dass man am Ende einen Datenwürfel oder eine dreidimensionale Datenmatrix erhält, in der zu jedem Pixel eines Flächensensors die mit den verschiedenen spektralen Kanälen aufgenommenen Bilder gespeichert sind. In der US 2019/0200848 A1 werden die hyperspektralen Daten dazu verwendet, augmentierte Bilder zu erzeugen, d.h. in der Qualität verbesserte stereoskopische Bilder im sichtbaren Bereich, die um Informationen aus den hyperspektralen Daten ergänzt wurden, um beispielsweise bestimmte Merkmale oder Strukturen im beobachteten Gewebe stärker hervorzuheben, die allein im sichtbaren Licht kaum bemerkbar wären.

Eine andere Möglichkeit, bestimmte Merkmale im Gewebe hervorzuheben, besteht in der Anregung und Detektion von Fluoreszenz. Ein optisches System, welches dies realisiert und beispielsweise als Endoskop ausgestaltet sein kann, beschreibt die WO 2015/023990 A1. Das optische System umfasst dazu zum einen eine hyperspektrale Kamera und zum anderen eine zusätzliche Lichtquelle, mit der Fluoreszenz angeregt werden kann. Damit lassen sich im Wechsel zwei Arten von Bildern aufnehmen: Zum einen lassen sich übliche hyperspektrale Bilder über sämtliche zur Verfügung stehenden Farbkanäle aufnehmen. In diesen Bildern werden die natürlichen Farben des Gewebes basierend auf deren Chromophoren wiedergegeben. Zum anderen lassen sich auf bestimmte Farbkanäle beschränkte Bilder aufnehmen, um beispielsweise Fluoreszenzsignale aufzunehmen. Als Beispiel wird der Farbstoff ICG (Indocyaningrün) genannt, der intravenös verabreicht wird und dazu verwendet werden kann, lebendes Gewebe zu markieren. Unter Bestrahlung mit Anregungslicht - im Wellenlängenbereich zwischen 600 nm und 900 nm -wird ICG zur Fluoreszenzemission im nahinfraroten Wellenlängenbereich zwischen 750 nm und 950 nm genutzt. Ein Emissionsmaximum für in Blut gelöstes ICG liegt bei etwa 830 nm. ICG wird beispielsweise in der Tumordiagnostik eingesetzt, um unter anderem den regionalen Zustand von Lymphknoten bei Tumoren minimal-invasiv zu untersuchen.

Mit dem in der US 2019/0200848 A1 beschriebenen Stereoendoskop lassen sich zwar augmentierte Stereobilder erzeugen, die einen höheren Informationsgehalt als rein im visuellen Bereich aufgenommene Bilder haben. In manchen Situationen wäre es jedoch wünschenswert, noch weitere Analyse- bzw. Abbildungsverfahren im Endoskop zur Verfügung zu haben, mit denen eine detailliertere Analyse des beobachteten Gewebes möglich ist.

### Beschreibung der Erfindung

Aufgabe der Erfindung ist es daher, ein Stereoendoskop zu entwickeln, welches gegenüber dem Stand der Technik verbesserte Möglichkeiten aufweist, augmentierte Bilder mit zusätzlichen Informationen darzustellen.

Diese Aufgabe wird für ein Stereoendoskop der eingangs beschriebenen Art durch folgende zusätzlichen Merkmale gelöst: In der ersten Sensoreinheit sind zwischen dem ersten Strahlteiler und dem ersten Sensor erste Manipulationsmittel angeordnet, mit denen der erste Lichtstrahl manipuliert wird. Analog sind in der zweiten Sensoreinheit zwischen dem zweiten Strahlteiler und dem vierten Sensor zweite, von den ersten Manipulationsmitteln verschiedene Manipulationsmittel angeordnet, mit denen der vierte Lichtstrahl manipuliert wird. In der Bildverarbeitungseinheit ist ein erster, auf die ersten Manipulationsmittel abgestimmter Bildverarbeitungsalgorithmus implementiert, sowie ein zweiter, auf die zweiten Manipulationsmittel abgestimmter Bildverarbeitungsalgorithmus. Der erste Bildverarbeitungsalgorithmus erzeugt ein erstes monoskopisches Bild und der zweite Bildverarbeitungsalgorithmus erzeugt ein zweites monoskopisches Bild. Dabei sind erste und zweite Manipulationsmittel, erster und vierter Sensor sowie erster und zweiter Bildverarbeitungsalgorithmus so vorgegeben, dass das erste und zweite monoskopisches Bild sowie das stereoskopische Bild komplementäre Bildinformationen enthalten.

Unter dem Begriff der "komplementären Bildinformationen" werden dabei Bildinformationen verstanden, die einander ergänzen. Das bedeutet, dass das erste monoskopische Bild über die Bildinformationen des stereoskopischen Bildes hinausgehende und in diesem nicht enthaltene Informationen enthält. Das zweite monoskopisches Bild wiederum enthält weitere Informationen, die im ersten monoskopisches Bild sowie im stereoskopischen Bild nicht enthalten sind. Verfahren, welche komplementäre Bildinformationen ergeben, sind somit grundsätzlich komplementär. Darüber hinaus können sich die komplementären Verfahren auch in anderen Eigenschaften unterscheiden, wie beispielsweise der Zeitdauer, die für die Aufnahme benötigt wird. So liefern beispielsweise hyperspektrale Analyseverfahren genaue Informationen, welche die Bestimmung von Gewebeparametern ermöglichen, sind jedoch vergleichsweise zeitaufwendig. Im Gegensatz dazu ist die Analyse von Fluoreszenzbildern - d.h. von Bildern einer Probe lebenden Gewebes, die zuvor mit Fluoreszenzmarkern markiert worden war - schnell und liefert - je nach Marker - spezifische Aussagen über die Lokalisierung einer Art von Gewebe in der Probe. Grundsätzlich hat ein Operateur durch die Bereitstellung mehrerer komplementärer Verfahren in einem Endoskop mehr Möglichkeiten zur Bildgebung, was eine Anpassung an eine Vielzahl verschiedener Patientensituationen ermöglicht; so kann beispielsweise Rücksicht auf die Unverträglichkeit bestimmter Medikamente genommen werden, etc.

Neben dem stereoskopischen Bild, was visuell dargestellt werden kann und in der Regel im sichtbaren Wellenlängenbereich aufgenommen wird, werden also mit zwei weiteren, zueinander komplementären Verfahren, monoskopisches Bilder aufgenommen. Die beiden Abbildungskanäle, die für das stereoskopische Bild zusammengeführt werden, sind also verschiedenen Untersuchungs- bzw. Abbildungsmethoden zugeordnet.

Üblicherweise erfordert jede Untersuchungs- bzw. Abbildungsmethode eigene Manipulationsmittel. Darunter werden im einfachsten Fall optische Elemente wie beispielsweise Filter oder Linsen verstanden, aber auch Prismen für eine spektrale Zerlegung oder komplexere Manipulationsmittel, wie sie beispielsweise für die Aufnahme hyperspektraler Bilder notwendig sind. Die Manipulationsmittel können bei komplexeren Methoden auch den zur Beleuchtung verwendeten Lichtstrahl manipulieren, beispielsweise durch Polarisation, wozu es zweckmäßig ist, die Beleuchtungsmittel so auszubilden, dass das Beleuchtungslicht ebenfalls über einen entsprechenden Strahlteiler, beispielsweise den ersten oder den zweiten Strahlteiler oder einen gesonderten Strahlteiler, aus der Sensoreinheit ausgekoppelt und in den Schaft, in welchem sich in der Regel ein Übertragungssystem befindet, eingekoppelt wird. In solchen Fällen kann die Sensoreinheit auch als Sensor- und Manipulationseinheit bezeichnet werden.

Im Stand der Technik stehen üblicherweise für die verschiedenen Aufnahme- bzw. Analysemethoden speziell angepasste Bildverarbeitungsalgorithmen zur Verfügung. Die aufgenommenen monoskopischen Bilder können dann mittels ebenfalls bekannter Bildverarbeitungsalgorithmen zur Verbesserung der Qualität des stereoskopischen Bildes verwendet werden, indem letzterem die Informationen aus ersterem überlagert werden. Dazu ist es nicht notwendig, auch diejenigen Bilder, die mittels der ersten und zweiten Manipulationsmittel erzeugt werden, stereoskopisch aufzunehmen. Mittels eines entsprechenden Bildverarbeitungsalgorithmus lässt sich sicherstellen, dass die Überlagerung mit den Informationen aus den monoskopischen Bildern den stereoskopischen Gesamteindruck des im visuellen Bereich aufgenommenen Bildes nicht zerstört.

Die Beleuchtungsmittel umfassen im einfachsten Fall eine einzige Lichtquelle, beispielsweise eine LED, bevorzugt jedoch mehrere Lichtquellen, die bevorzugt auf die Manipulationsmittel abgestimmt sind. Soll beispielsweise Fluoreszenz angeregt werden, muss die verwendete Lichtquelle Licht abstrahlen, mit dem die Fluoreszenz angeregt werden kann. Dazu kann beispielsweise eine inkohärentes Licht abstrahlende LED verwendet werden. Bevorzugt umfassen die Beleuchtungsmittel mindestens eine erste und eine zweite Lichtquelle und mindestens eine der Lichtquellen strahlt bevorzugt monochromatisches und kohärentes Licht ab, ist also als Laserlichtquelle ausgebildet. Die Verwendung von Laserlichtquellen gibt Zugriff auf zahlreiche weitere Analysemethoden.

Beispielhaft sind die ersten und zweiten Manipulationsmittel, der erste und vierte Sensor und der erste und zweite Bildverarbeitungsalgorithmus alternativ ausgebildet zur Erzeugung (i.) eines Fluoreszenzbildes aus Fluoreszenzsignalen, (ii.) eines hyperspektralen Bildes, (iii.) eines polarisationsaufgelösten Bildes, (iv.) eines Raman-spektroskopischen Bildes, (v.) eines optisch-kohärenztomographischen Bildes, (vi.) eines Speckle-Kontrast-Bildes, (vii.) eines Multiband-Bildes, oder (viii.) eines Narrowband-Bildes.

Für die Erzeugung eines Raman-spektroskopischen Bildes müssten die entsprechenden Manipulationsmittel ein auf entsprechend höhere Wellenlängen optimiertes Spektrometer umfassen. Im Falle eines Narrowband-Bildes würde eine Lichtquelle verwendet werden, die Licht in einem oder mehreren schmalbandigen Spektralbereichen abstrahlt; für die Detektion würden die Manipulationsmittel vor dem Bildsensor einen Bandpassfilter umfassen. Beide Maßnahmen können alternativ oder in Kombination eingesetzt werden.

Zur Erzeugung eines polarisationsaufgelösten Bildes lassen sich mehrere Mittel verwenden. Beispielsweise kann die entsprechende Lichtquelle mit einem Polarisator versehen sein. Wird das Licht über eine Lichtleitfaser zum distalen Ende geleitet, so kann der Polarisator beispielsweise am Austrittspunkt des Lichtes aus der Lichtleitfaser angeordnet sein. Gegebenenfalls kann auch mit unpolarisiertem Licht beleuchtet werden. Für die Detektion können Flächendetektoren mit vorgeschalteten Polarisationsfiltern verwendet werden. Eine andere Möglichkeit besteht darin, den Flächendetektor so auszugestalten, dass er ein Polarisationsmuster aufweist. Eine Gruppe von Pixeln kann dabei den Polarisationszustand bestimmen, ähnlich zu der Farbbestimmung durch Bayer-Muster bei einer normalen Kamera, welche in den Farben rot, grün und blau detektiert.

Für die Erzeugung eines Speckle-Kontrast-Bildes kann für die Beleuchtung eine außerhalb des sichtbaren Spektrums liegende Laserwellenlänge verwendet werden; in diesem Fall kann die Bildgebung simultan mit der im visuellen Bereich für das stereoskopische Bild erfolgen.

Um ein optisch-kohärenztomographisches Bild aufnehmen zu können, wird eine entsprechende Sensoreinheit verwendet, welche unter anderem ein Interferometer umfasst, wo das vom Objekt zurückgestreute Licht mit dem Beleuchtungslicht überlagert wird. Eine kohärente Beleuchtung lässt sich über die abbildende Optik realisieren. Die diversen Scanner des OCT-Moduls müssten dabei nicht in der Sensoreinheit angeordnet sein, sondern könnten beispielsweise im Endoskop-Körper, synonym auch Endoskop-Gehäuse, untergebracht sein. Die Verbindung zu der kohärenten Lichtquelle und zum Interferometer würde über Lichtleitfaser erfolgen. Die Lichtquelle kann dabei außerhalb des Endoskops angeordnet sein.

Zweckmäßig sind der erste und der zweite Strahlteiler unterschiedlich ausgebildet, um eine höhere Flexibilität bei dem Einsatz verschiedener Methoden bzw. Manipulationsmittel zu erreichen.

In einer besonders bevorzugten Ausgestaltung sind die ersten Manipulationsmittel, der erste Sensor und der erste Bildverarbeitungsalgorithmus zur Erzeugung eines Fluoreszenzbildes aus Fluoreszenzsignalen ausgebildet und der erste Sensor ist für die Detektion des entsprechenden Fluoreszenzspektrums ausgelegt. Beispielsweise lässt sich als Marker ICG verwenden; in diesem Fall müssen die ersten Manipulationsmittel, der erste Sensor und der erste Bildverarbeitungsalgorithmus Fluoreszenzsignale im Infraroten verarbeiten. Da die Fluoreszenz von ICG außerhalb des sichtbaren Bereichs liegt, wird das im sichtbaren Wellenlängenbereich aufgenommene stereoskopische Bild nicht gestört. Eine Überlagerung ist dennoch möglich, wenn im Nachhinein die Bereiche, von denen Fluoreszenz ausgeht, in Falschfarben dargestellt werden. Die Überlagerung kann ohne Tiefenauflösung erfolgen, dann wird auf jedem der beiden Kanäle oder auch nur auf einem der Kanäle die gleiche Information für ICG dargestellt. Anhand der optischen Daten sowie der Tiefeninformationen aus der stereoskopischen Aufnahme lässt sich dieses monoskopische Bild jedoch auch in ein stereoskopisches Bild umrechnen. Auf diese Weise lassen sich die vorhandenen, verschiedenen Gewebearten besser differenzieren, sodass besser eingeschätzt werden kann, in welchem Umfang beispielsweise ein Tumor entfernt werden muss. Anstelle von ICG lassen sich selbstverständlich auch andere Fluoreszenzmarker, beispielsweise Methylen-Blau, mit darauf abgestimmten Manipulationsmitteln und Sensoren verwenden, auch auf diese Weise kann zu einer verbesserten Differenzierung verschiedener Gewebearten beigetragen werden.

Besonders bevorzugt sind die zweiten Manipulationsmittel, der vierte Sensor und der zweite Bildverarbeitungsalgorithmus zur Erzeugung eines hyperspektralen Bildes ausgelegt; die zweiten Manipulationsmittel umfassen zu diesem Zweck ein hyperspektrales Spektrometer. Durch die Berücksichtigung von Aufnahmen im Spektralbereich außerhalb des sichtbaren Spektrums lassen sich die Aufnahmen innerhalb des sichtbaren Spektrums, also das stereoskopische Bild, optimieren, indem weitere Bildverarbeitungsalgorithmen, beispielsweise zur Kontrasterhöhung oder zur Verstärkung bei wenig Licht, eingesetzt werden. Bevorzugt wird zur Erzeugung des hyperspektralen Bildes das Verfahren des spektralen Scannens benutzt. Dabei werden für die verschiedenen, sehr schmalbandigen Wellenlängenbereiche nacheinander monochromatische oder nahezu monochromatische Bilder aufgenommen, zwischen den Aufnahmen wird beispielsweise ein durchstimmbarer optischer Bandpassfilter auf den nächsten Wellenlängenbereich eingestellt. Dies ermöglicht einen relativ kompakten Aufbau der hyperspektralen Kamera in der Sensoreinheit. Alternativ lassen sich aber auch andere bekannte Verfahren zur Erzeugung eines hyperspektralen Bildes verwenden, beispielsweise das Schnappschuss-Verfahren oder das räumliche Scannen.

Bevorzugt sind der erste und der zweite Strahlteiler im visuellen Spektralbereich teilweise reflektierend und im nahen Infrarot überwiegend transmittierend ausgebildet. Auch die umgekehrte Ausgestaltung mit überwiegender Transmission im Sichtbaren und teilweiser Reflexion im nahen Infraroten ist denkbar, je nach Herstellungsverfahren. Insbesondere für den Fall, dass ICG als Fluoreszenzmarker verwendet wird, ist der erste Strahlteiler für einen ersten Wellenlängenbereich zwischen 650 nm und 900 nm mindestens teilweise transparent, wobei hingegen der Transmissionsgrad für Wellenlängen zwischen 400 nm und 650 nm weniger als 10 % beträgt. Besonders bevorzugt beträgt der Transmissionsgrad des ersten Strahlteilers für Wellenlängen zwischen 400 nm und 650 nm weniger als 0,5 % und für Wellenlängen zwischen 750 nm und 900 nm mehr als 95 %.

Insbesondere in dem Fall, dass der zweite Strahlteiler mit einem Hyperspektrometer verwendet wird, weist der zweite Spektralteiler für Wellenlängen zwischen 700 nm und 1100 nm einen Transmissionsgrad von mehr als 90 %, bevorzugt nahezu 100 % auf, wobei hingegen das Verhältnis von Transmission zu Reflexion im Wellenlängenbereich zwischen 400 nm und 600 nm konstant ist und beispielsweise zwischen 85:15 und 30:70, beispielsweise bei 50:50 liegen kann.

Dies ermöglicht beispielsweise, dass die erste Sensoreinheit mit den ersten Manipulationsmitteln, dem ersten Sensor und dem ersten Bildverarbeitungsalgorithmus für die Detektion des Fluoreszenzspektrums von ICG sowie die Erzeugung eines entsprechenden Fluoreszenzbildes verwendet werden kann. Neben dem stereoskopischen Bild werden so ein Fluoreszenzbild des Fluoreszenzspektrums von ICG sowie ein hyperspektrales Bild erzeugt. Die letztgenannten beiden Bilder sind monoskopisch und werden über weitere Bildverarbeitungsalgorithmen zur Verbesserung der Qualität in der Darstellung des stereoskopischen Bildes oder zur Darstellung zusätzlicher, komplementärer Informationen verwendet. Die Darstellung der komplementären Informationen kann dem stereoskopischen Bild überlagert werden, sie kann aber auch getrennt davon erfolgen.

Der für den visuellen Bereich abgespaltene Strahlungsanteil ist vorzugsweise geringer als derjenige, der für das Fluoreszenzbild im nahen Infrarot bzw. für das hyperspektrale Bild abgespalten wird. Insbesondere für das hyperspektrale Bild wird das zur Verfügung stehende Licht spektral aufgespalten, weshalb in diesem Fall ein höherer Strahlungsanteil benötigt wird. Als Strahlteiler lassen sich übliche dichroitische Strahlteiler verwenden, beispielsweise Glasscheiben, die in einem Winkel von 45° in den Strahlengang eingebracht werden. Alternativ lassen sich auch zwei Prismen verwenden. Das Teilungsverhältnis des Lichtstrahls ist abhängig von der Dicke der Beschichtung sowie von der Wellenlänge.

In einer weiteren Ausgestaltung ist zwischen mindestens einem der beiden Strahlteiler und einem der diesem Strahlteiler zugeordneten Sensoren mindestens ein weiterer Strahlteiler angeordnet, der Licht in einen weiteren Lichtstrahl auf einen weiteren Sensor ablenkt. Zwischen dem weiteren Sensor und dem weiteren Strahlteiler sind außerdem weitere Manipulationsmittel angeordnet. Auf diese Weise lassen sich weitere Analyse- bzw. Bildgebungsverfahren integrieren, gegebenenfalls in Form einer Kaskade von Strahlteilern. Dabei sollten die Verfahren, die mit Licht geringerer Intensität auskommen, am Ende der Kaskade durchgeführt werden.

Wie vorangehend bereits beschrieben, ist die Bildverarbeitungseinheit dazu ausgelegt, bevorzugt Daten des ersten und zweiten monoskopisches Bildes zur Verbesserung der Qualität des stereoskopischen Bildes zu verwenden. Alternativ oder in Ergänzung kann auch ein drittes monoskopisches Bild erzeugt werden, welches einen anderen Blick auf das Untersuchungsobjekt ermöglicht. Unter einer Qualitätsverbesserung wird insbesondere verstanden, dass das Stereobild zusätzliche, zum visuellen Bild komplementäre Informationen aufzeigt. Aus einem mit Fluoreszenzsignalen erzeugten monoskopischen Bild des einen Kanals kann unter Zuhilfenahme der beiden Einzelbilder für das stereoskopische Bild ein weiteres monoskopisches Fluoreszenzbild für den anderen Kanal berechnet werden, so dass dann auch eine stereoskopische Darstellung der Fluoreszenz möglich ist. Gleiches gilt auch für die mit anderen Verfahren erzeugten Bilder, beispielsweise das hyperspektrale Bild. Dies trägt zur verbesserten Unterscheidung und zur verbesserten Zustandserkennung der verschiedenen Gewebearten bei; es lassen sich beispielsweise Rückschlüsse auf Tumorbefall, Durchblutung, Sauerstoffsättigung, Hämoglobingehalt, etc. ziehen, da sich anhand der Fluoreszenzmarkierung sowie der hyperspektralen Analyse verschiedene Eigenschaften oder Parameter erfassen lassen, die in verschiedenen Gewebearten unterschiedlich vorhanden sind. Beispielsweise lassen sich die Gewebeoxygenierung (StO₂), der Perfusionsindex im nahen Infrarot, der Gewebe-Hämoglobin-Index (Tissue Haemoglobin Index, THI), der Gewebe-Wasser-Index (Tissue Water Index, TWI) oder der Gewebe-Fett-Index (Tissue Lipid Index, LPI) erfassen; die verschiedenen Gewebearten können auf Basis der Bildanalyse unterschiedlich visualisiert werden.

Die erste Sensoreinheit und/oder die zweite Sensoreinheit können bevorzugt als Steckmodule ausgebildet sein, und je nach verwendeter Analysemethode bzw. Manipulationsmethode in den entsprechenden Endoskop-Körper eingesetzt werden.

Das vorangehend beschriebene Stereoendoskop lässt sich bevorzugt zur Erzeugung verbesserter stereoskopischer Bilder von Gewebeproben verwenden, wobei ein erstes monoskopisches Bild erzeugt wird, indem Fluoreszenzsignale im Infrarotbereich detektiert werden, bevorzugt solche, die von dem Farbstoff ICG erzeugt werden, und ein zweites monoskopisches Bild erzeugt wird, indem ein hyperspektrales Bild detektiert wird, wobei erstes und zweites monoskopisches Bild bei einer Bildverarbeitung zur Erhöhung des Kontrasts und/oder zur Verstärkung der Intensität bei dem stereoskopischen Bild verrechnet werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Es zeigen:
- Fig. 1: den grundsätzlichen Aufbau eines Stereoendoskops,
- Fig. 2: eine erste Ausgestaltung eines Stereoendoskops,
- Fig. 3: eine zweite Ausgestaltung eines Stereoendoskops und
- Fig. 4: die zweite Ausgestaltung mit dem Verlauf von Lichtwegen.

### Ausführliche Beschreibung der Zeichnungen

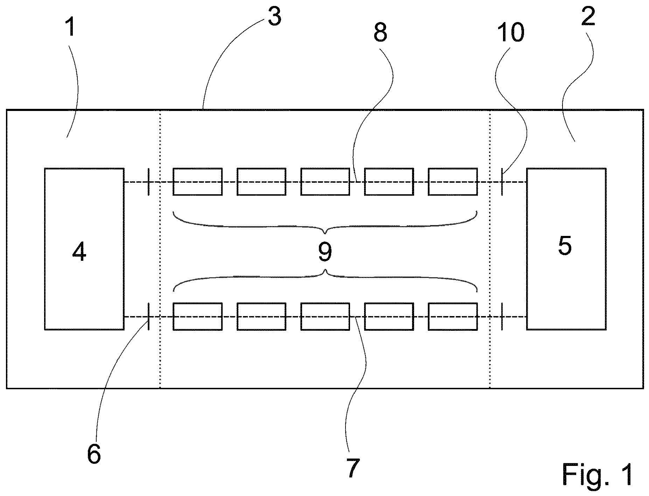

Fig. 1 zeigt den grundsätzlichen Aufbau eines Stereoendoskops, wie er im Prinzip auch aus dem Stand der Technik bekannt ist. Das Stereoendoskop gliedert sich in ein distales Ende 1, ein proximales Ende 2 und einen Schaft 3, der das distale Ende 1 mit dem proximalen Ende 2 verbindet. Die körperliche Trennung der Bereiche ist in Fig. 1 durch die senkrechten, gestrichelten Linien angedeutet. Neben einem Eingangsobjektiv 4 sind im distalen Ende 1 auch - hier nicht gezeigte - Lichtquellen oder zumindest Lichtaustrittsflächen angeordnet, die eine Beleuchtung des zu untersuchenden Objekts ermöglichen. Im proximalen Ende 2 ist ein optisches Abbildungssystem 5 angeordnet, im einfachsten Fall beispielsweise in Form eines stereoskopischen Kameraobjektivs. Das Eingangsobjektiv 4 bildet das Objekt in einer ersten Zwischenbildebene 6 ab, wobei die Abbildung bereits stereoskopisch erfolgt, d. h. in einen ersten optischen Abbildungskanal 7 und einen zweiten optischen Abbildungskanal 8. Die beiden optischen Kanäle 7, 8 sind hier durch die waagerechten, gestrichelten Linien gekennzeichnet. Im Schaft 3, welcher vorzugsweise starr ausgebildet ist, befindet sich ein optisches Übertragungssystem 9, welches ein Zwischenbild von der ersten Zwischenbildebene 6 in eine zweite Zwischenbildebene 10 abbildet. Von dort wird es mittels des optischen Abbildungssystems 5 zur weiteren Verwendung bzw. Auswertung oder Manipulation auf weitere optische Elemente, die hier nicht gezeigt sind, abgebildet. Im Falle eines stereoskopischen Kameraobjektivs erfolgt die Abbildung auf Flächendetektoren.

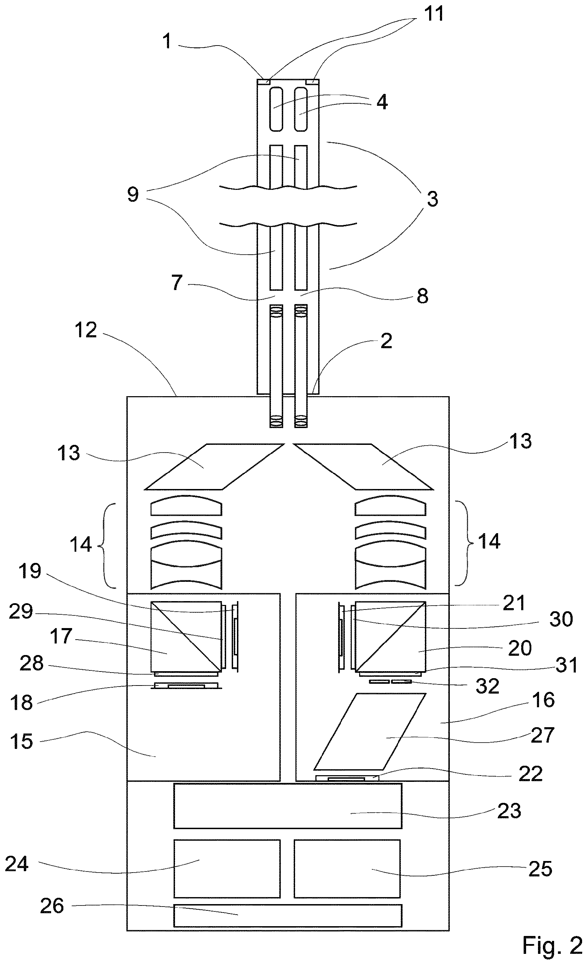

In der in Fig. 2 gezeigten Konfiguration werden unterschiedliche Objektive 4 verwendet und jedes der Bilder wird in einem eigenen Kanal - dem ersten optischen Abbildungskanal 7 und dem zweiten optischen Abbildungskanal 8 - vom distalen Ende 1 zum proximalen Ende 2 übertragen. Die Übertragung kann aber auch in einem einzigen Kanal erfolgen, wobei dann entweder mittels zweier Objektive 4 zwei Bilder abgebildet werden oder mittels eines einzigen Objektivs zwei Bilder überlagert abgebildet und übertragen werden und eine Trennung in zwei optische Abbildungskanäle mit zwei einzelnen Bildern durch eine Pupillenteilung erst am Ende des Übertragungssystems 9 im optischen Abbildungssystem 5 erfolgt.

Das stereoskopische Bild im visuellen Bereich, welcher Wellenlängen im Bereich von etwa 380 nm bis zu 780 nm umfasst, zeigt zwar wichtige Informationen und verschafft dem Operateur einen guten Überblick über das zu behandelnde Gewebe. Jedoch lassen sich mit speziellen Methoden, beispielsweise der Markierung von Gewebe mit fluoreszierenden Farbstoffen, noch detailliertere Informationen sichtbar machen, die dem Operateur eine genauere Planung des Eingriffs sowie eine bessere Eingrenzung beispielsweise des zu entnehmenden, von bösartigen Tumoren befallenen Gewebes ermöglichen.

Die im Folgenden vorgestellten Stereoendoskope ermöglichen es, zusätzlich zum stereoskopischen Bild im visuellen Bereich, weitere monoskopische Bilder, welche zum stereoskopischen Bild komplementäre Bildinformationen enthalten, aufzunehmen bzw. zu erzeugen und gegebenenfalls mit dem stereoskopischen Bild zu verrechnen, um eine Darstellung aller Bildinformationen in einem Bild zu erhalten. Unter dem Begriff der "komplementären Bildinformationen" werden dabei Bildinformationen verstanden, die im ursprünglichen stereoskopischen Bild nicht sichtbar waren, sei es aufgrund der einfachen Abbildung ohne Manipulation oder aufgrund des eingeschränkten spektralen Bereichs. Die komplementären Bildinformationen ergänzen die Bildinformationen des stereoskopischen Bildes, sind jedoch nicht redundant. Verfahren, welche komplementäre Bildinformationen ergeben, sind somit grundsätzlich komplementär.

Fig. 2 zeigt eine erste Ausgestaltung eines Stereoendoskops. Identische Bezugszeichen korrespondieren zu gleichen Elementen wie in anderen Figuren und werden ggf. nicht noch einmal erläutert. Am distalen Ende 1 des Schaftes 3 sind hier zusätzlich Beleuchtungsmittel 11 gezeigt. Es kann sich dabei um Leuchtmittel handeln, beispielsweise LED, die direkt am distalen Ende 1 eingesetzt sind. Vorteilhaft handelt es sich jedoch um die Enden von Lichtleitfaser, aus denen Licht ausgekoppelt wird. Dies ermöglicht es, die Leuchtmittel außerhalb des Schaftes 3 anzuordnen, was einen leichteren Wechsel bei Ausfall oder zu einer anderen Beleuchtung ermöglicht. Darüber hinaus wird auch die Wärmebildung am distalen Ende 1 verhindert. Schließlich ermöglicht dies auch die Verwendung größerer Lichtquellen, beispielsweise von Lichtquellen, die monochromatisches, kohärentes Licht abstrahlen.

Am proximalen Ende 2 des Schaftes 3 ist eine Analyseeinheit 12 angeordnet, die beispielsweise in den Körper oder das Gehäuse des Endoskops integriert sein kann. Der Schaft 3 einschließlich der darin verwendeten optischen Elemente kann beispielsweise dem in der DE 10 2017 113 274 A1 der Anmelderin beschriebenen entsprechen. Das optische Übertragungssystem 9 bzw. das in Fig 2 der Übersichtlichkeit halber nicht gesondert dargestellte Abbildungssystem 5 bildet im vorliegenden Fall das Licht, welches vom distalen Ende 1 über den Schaft 3 zum proximalen Ende 2 übertragen wird, nicht auf Flächendetektoren einer Kamera ab, sondern auf Prismen 13, welche die Lichtstrahlen so zueinander versetzen, dass der Abstand zwischen ihnen größer wird, um mehr Platz für Manipulationen an den Lichtstrahlen zu erhalten. Ausgangsseitig der Prismen 13 werden die Lichtstrahlen auf Adapteroptiken 14, die hier beispielhaft durch eine Kombination von Linsen dargestellt werden, auf die jedoch die Ausgestaltung der Adapteroptiken 14 nicht beschränkt ist, gelenkt. Licht aus dem ersten Abbildungskanal 7 wird über eine der Adapteroptiken 14 in eine erste Sensoreinheit 15 abgebildet. Licht aus dem zweiten Abbildungskanal 8 wird über die andere der Adapteroptiken 14 in eine zweite Sensoreinheit 16 abgebildet.

Die erste Sensoreinheit 15 umfasst einen ersten Strahlteiler 17, welcher das Licht in einen ersten Lichtstrahl 41 und in einen zweiten Lichtstrahl 42 aufteilt. Die erste Sensoreinheit 15 umfasst außerdem einen ersten Sensor 18 und einen zweiten Sensor 19. Der erste Sensor 18 detektiert Licht des ersten Lichtstrahls 41 und wandelt dieses in erste elektromagnetische Signale um; der zweite Sensor 19 detektiert Licht des zweiten Lichtstrahls 42 und wandelt dieses in zweite elektromagnetische Signale um.

Die zweite Sensoreinheit 16 umfasst einen zweiten Strahlteiler 20, welcher das Licht in einen dritten Lichtstrahl 43 und in einen vierten Lichtstrahl 44 aufteilt. Die zweite Sensoreinheit 16 umfasst außerdem einen dritten Sensor 21, welcher Licht des dritten Lichtstrahls 43 detektiert und in dritte elektromagnetische Signale umwandelt, sowie einen vierten Sensor 22, welcher Licht des vierten Lichtstrahls 44 detektiert und in vierte elektromagnetische Signale umwandelt.

Der zweite Sensor 19 und der dritte Sensor 21 sind im hier gezeigten Beispiel ohne Beschränkung der Allgemeinheit gleichartig und detektieren Licht im visuellen Bereich, d. h. im für das menschliche Auge sichtbaren Spektralbereich zwischen 380 nm und etwa 780 nm. Eine Zuordnung zu oder Kombination von anderen Pfaden ist grundsätzlich ebenfalls möglich. Die ersten bis vierten elektromagnetischen Signale werden in einer Bildverarbeitungseinheit 23 verarbeitet, wobei die zweiten und dritten elektromagnetischen Signale zu einem stereoskopischen Bild verarbeitet werden.

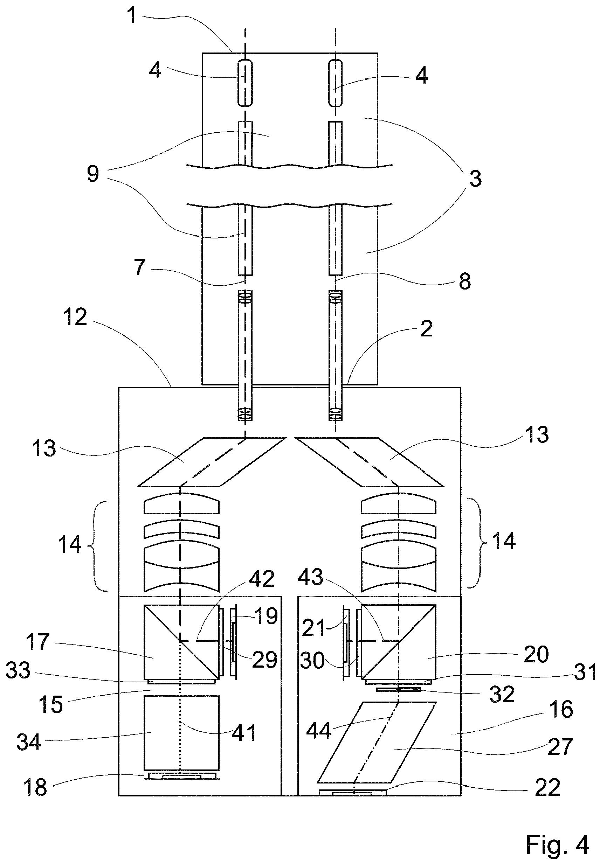

Die Lichtwege und Lichtstrahlen sind in Fig. 4, die einen Ausschnitt aus einem Endoskop zeigt, im Detail dargestellt. Der zweite und dritte Lichtstrahl 42, 43 sind, da sie auf gleichartige Detektoren treffen, beide gestrichelt gezeichnet. Der erste Lichtstrahl 41 ist gepunktet dargestellt, der vierte Lichtstrahl 44 mit einer Strichpunkt-Linie.

Um möglichst viele komplementäre Informationen des Untersuchungsobjekts zu erhalten, werden der erste Lichtstrahl 41 und der vierte Lichtstrahl 44 auf voneinander verschiedene Weisen manipuliert bzw. analysiert. Die Methoden, mit der diese beiden Lichtstrahlen manipuliert bzw. analysiert werden, unterscheiden sich voneinander und werden so ausgewählt, dass sie zum stereoskopischen Bild und zueinander komplementäre Bildinformationen liefern. Vorteilhaft weisen dazu auch der erste Strahlteiler 17 und der zweite Strahlteiler 20 verschiedene Eigenschaften auf. Dies ist jedoch nicht zwingend notwendig, wenn die Methoden, mit denen der erste Lichtstrahl 41 und der vierte Lichtstrahl 44 manipuliert bzw. analysiert werden, auch bei gleichartig ausgestalteten Strahlteilern komplementäre Bildinformationen liefern.

Dementsprechend sind in der ersten Sensoreinheit 15 zwischen dem ersten Strahlteilern 17 und dem ersten Sensor 18 erste Manipulationsmittel angeordnet, mit denen der erste Lichtstrahl 41 manipuliert wird, und zwischen dem zweiten Strahlteiler 20 und dem vierten Sensor 22 zweite Manipulationsmittel, mit denen der vierte Lichtstrahl 44 manipuliert wird. Erste Manipulationsmittel und zweite Manipulationsmittel sind dabei voneinander verschieden. Im einfachsten Fall umfassen die Manipulationsmittel einfache Filter, mit denen beispielsweise das sichtbare Licht ausgeblendet wird und nur Licht im nahen Infrarot, im fernen Infrarot und/oder im ultravioletten Spektralbereich transmittiert wird.

Zusätzlich sind in der Bildverarbeitungseinheit 23 entsprechende, auf die Manipulationsmittel abgestimmte, Bildverarbeitungsalgorithmen implementiert. Konkret sind ein erster, auf die ersten Manipulationsmittel abgestimmter Bildverarbeitungsalgorithmus implementiert, sowie ein zweiter, auf die zweiten Manipulationsmittel abgestimmter Bildverarbeitungsalgorithmus. Der erste Bildverarbeitungsalgorithmus erzeugt ein erstes monoskopisches Bild, der zweite Bildverarbeitungsalgorithmus erzeugt ein zweites monoskopisches Bild. Erster und zweiter Bildverarbeitungsalgorithmus, erste und zweite Manipulationsmittel sowie erster und vierter Sensor 18, 22 sind dabei so vorgegeben, dass das erste und zweite monoskopische Bild sowie das stereoskopische Bild komplementäre Bildinformationen enthalten. Erster und vierter Sensor 18, 22 können dabei auch gleich sein, wenn die Manipulationsmittel bzw. Bildverarbeitungsalgorithmen dennoch die Bedingung erfüllen, dass komplementäre Bildinformationen erzeugt werden.

Die Bildinformationen des stereoskopischen Bildes können dann um die Bildinformationen des einen oder der beiden monoskopischen Bilder ergänzt werden, sodass man ein augmentiertes Bild erhält, welches dank der komplementären Informationen einen deutlich höheren Informationsgehalt aufweist, als das stereoskopische Bild für sich genommen. Die Ergänzung der Informationen bzw. die Überlagerung aller Bildinformationen kann ebenfalls in der Bildverarbeitungseinheit 23 vorgenommen werden. Allgemein ist die Bildverarbeitungseinheit 23 dazu ausgelegt, Daten des ersten und des zweiten monoskopischen Bildes zur Verbesserung der Qualität des stereoskopischen Bildes zu verwenden bzw. zu verrechnen, oder aber ein drittes monoskopisches Bild zu erzeugen. Sie kann innerhalb des Körpers oder Gehäuses des Endoskops angeordnet sein. Vorteilhaft ist sie jedoch außerhalb und räumlich getrennt vom Endoskop angeordnet, um einerseits den Bauraum des Endoskops klein zu halten und andererseits die Wärmeentwicklung im Bereich des Endoskops zu reduzieren.

Zusätzlich befinden sich in der Analyseeinheit 12 weitere Elemente: eine erste Steuerungseinheit 24, mit welcher die erste Sensoreinheit gesteuert wird und eine zweite Steuerungseinheit 25, mit welcher die zweite Sensoreinheit gesteuert wird. Auch die Steuerungseinheiten 24 und 25 können wie die Bildverarbeitungseinheit 23 sowohl innerhalb als auch vorteilhaft außerhalb des Gehäuses angeordnet sein. Zusätzlich kann über diese Steuerungseinheiten 24 und 25 auch eine Steuerung der Beleuchtung erfolgen, wenn beispielsweise auf beide Abbildungskanäle 7 und 8 zusätzlich zu dem sichtbaren Licht noch Licht anderer Wellenlängen eingekoppelt werden soll, wobei dieses zusätzliche Licht für die beiden Abbildungskanäle 7 und 8 verschieden ist. Außerdem befindet sich in der Analyseeinheit 12 noch ein Anschlussinterface 26, welches unter anderem zur Stromversorgung dient. Zusätzlich dient es noch zum Transfer von Daten und Steuersignalen und kann auch zur Einkopplung von Licht dienen, wenn die Lichtquellen beispielsweise außerhalb der Analyseeinheit 12 oder nicht am distalen Ende 1 des Schaftes 3 angeordnet sind, was beispielsweise dann der Fall sein wird, wenn die Beleuchtungsmittel mehrere, mindestens 2 Lichtquellen umfassen und mindestens eine der Lichtquellen monochromatisches, kohärentes Licht abstrahlend ausgebildet ist.

In der hier gezeigten Ausführung sind die Bildverarbeitungseinheit 23 sowie die Steuerungseinheiten 24 und 25 zwar in der Analyseeinheit 12 angeordnet, diese Anordnung ist jedoch nicht zwingend. Sowohl die Steuerungseinheiten 24 und 25 als auch die Bildverarbeitungseinheit 23 können auch außerhalb der Analyseeinheit 12 angeordnet sein. Insbesondere kann die Bildverarbeitungseinheit 23 auch Teil eines in der Peripherie eines robotischen Operationssystems angeordneten Computers sein. Die erste Steuerungseinheit 24 und die zweite Steuerungseinheit 25 sind hier als getrennte Einheiten gezeigt, können aber ebenfalls in einer Einheit zusammengefasst werden, wobei die Steuerungseinheit dann über verschiedene Ausgänge mit der ersten Sensoreinheit 15 und der zweiten Sensoreinheit 16 verbunden wäre. Die erste Sensoreinheit 15 und die zweite Sensoreinheit 16 können in einer bevorzugten Ausgestaltung als Steckmodule ausgebildet sein, so dass ein Wechsel zwischen verschiedenen Manipulationsmethoden bzw. Analysemethoden erleichtert wird, in dem nur die Steckmodule in der Analyseeinheit 12 ausgetauscht werden müssen. Die Steckmodule können mit Kennungen versehen sein, so dass die Bildverarbeitungseinheit 23 über die Steuerungseinheiten 24 bzw. 25 automatisch erkennt, welches Steckmodul sich in der Analyseeinheit 12 befindet und einen entsprechenden Bildverarbeitungsalgorithmus auswählt.

Erste und zweite Manipulationsmittel, erster Sensor 18 und vierter Sensor 22 sowie erster und zweiter Bildverarbeitungsalgorithmus sind also zur Erzeugung von Bildinformationen ausgelegt, die zueinander sowie zum Informationsgehalt des stereoskopischen Bildes komplementär sind. Insbesondere können sie alternativ ausgebildet sein zur Erzeugung eines Fluoreszenzbildes aus Fluoreszenzsignalen im Infrarotbereich, eines hyperspektralen Bildes, eines polarisationsaufgelösten Bildes, eines Raman-spektroskopischen Bildes, eines optisch-kohärenztomographischen Bildes, eines Speckle-Kontrast-Bildes, eines Multiband-Bildes oder eines Narrowband-Bildes.

Bei dem in Fig. 2 dargestellten Gerät sind die ersten Manipulationsmittel, der erste Sensor 18 und der erste Bildverarbeitungsalgorithmus zur Erzeugung eines Fluoreszenzbildes aus Fluoreszenzsignalen im Infrarotbereich ausgebildet. Die zweiten Manipulationsmittel, der vierter Sensor 22 und der zweite Bildverarbeitungsalgorithmus sind hingegen zu Erzeugung eines hyperspektralen Bildes ausgebildet. Die zweiten Manipulationsmittel umfassen zu diesem Zweck ein hyperspektrales Spektrometer 27. Zur verbesserten Erzeugung von komplementären Informationen sind hier auch der erste Strahlteiler 17 und der zweite Strahlteiler 20 unterschiedlich ausgebildet. Der erste Strahlteiler 17 wird dabei so gewählt, dass er für die Fluoreszenzsignale in der hier gezeigten Konfiguration eine möglichst hohe Transmission aufweist. Welche Fluoreszenzsignale detektiert werden sollen, hängt selbstverständlich von den verwendeten Fluoreszenzmarkern ab. Ohne Beschränkung der Allgemeinheit soll der erste Sensor hier für die Detektion des Fluoreszenzspektrums von ICG ausgelegt sein. Die Emissionswellenlänge von ICG hat ein Maximum bei 830 nm, der erste Sensor 18 ist daher so ausgelegt, dass er elektromagnetischer Strahlung dieser Wellenlänge detektieren kann. Der erste Lichtstrahl 41 wird hier zusätzlich durch einen ersten Filter 28 gefiltert, welcher für die Emissionswellenlänge von ICG ausgelegt ist und Licht beispielsweise im Wellenlängenbereich von 810 nm bis 850 nm durchlässt. Beispielsweise kann der Filter Semrock FF01-832/3725 verwendet werden. Vor dem zweiten Sensor 19 ist ein zweiter Filter 29 angeordnet, welcher Licht außerhalb des sichtbaren Bereichs, d. h. ultraviolettes Licht und Infrarotlicht blockt. Beispielsweise kann der Filter Edmund #89-794 verwendet werden. Vor dem dritten Sensor 21 ist ein dritter Filter 30 angeordnet, welcher zum zweiten Filter 29 identisch aufgebaut ist. Grundsätzlich ist die Verwendung von Filtern optional. Je nach verwendeter Methode reicht die Beschichtung des Strahlteilers ggf. auch aus, um eine entsprechende spektrale Trennung - die einer Filterung entspricht - des Lichts vorzunehmen.

Wie bereits weiter oben erwähnt, wird als weiteres komplementäres Verfahren unter Verwendung der zweiten Sensoreinheit 16 eine hyperspektrale Analyse des vierten Lichtstrahls 44 vorgenommen, wozu es vorteilhaft ist, dass der zweite Strahlteiler 20 nicht nur im nahinfraroten Spektralbereich von mehr als 700 nm einen hohen Transmissionsgrad von mehr als 90 % aufweist, sondern auch im sichtbaren Spektralbereich einen hohen Transmissionsgrad von bevorzugt mehr als 50 % bis zu 80 % aufweist. Dies liegt darin begründet, dass das zur Verfügung stehende Licht im hyperspektralen Spektrometer 27 spektral aufgespalten wird und somit für jeden Wellenlängenbereich effektiv viel weniger Intensität zur Verfügung steht, weshalb hier ein höherer Strahlungsanteil benötigt wird.

Ausgangsseitig des Strahlteilers 20 ist zwischen diesem und dem Hyperspektrometer zunächst ein Gelbfilter 31, beispielsweise der Filter Schott GG495, angeordnet. Der Filter dient hier im Zusammenhang mit der hyperspektralen Analyse über ein Gitter dazu, höhere Wellenlängen zu unterdrücken und somit höhere Ordnungen im Gitter-Spektrometer auszublenden. Nach Passieren des Filters tritt das Licht durch einen Spalt 32 in das hyperspektrale Spektrometer 27.

Die beiden Strahlteiler 17 und 20 können als einfache, dichroitisch wirkende Glasscheiben ausgestaltet sein. Im vorliegenden Beispiel sind sie jedoch jeweils würfel- oder quaderförmig aus zwei Prismen zusammengesetzt, was für die Vermeidung von Abbildungsfehlern vorteilhaft ist: Sowohl der erste Strahlteiler 17 als auch der zweite Strahlteiler 20 sind auf derjenigen Seite, wo das Licht einfällt, mit einer Antireflexionsschicht für den Wellenlängenbereich zwischen 400 nm und 1100 nm beschichtet. Dies beugt Lichtverlust vor. Auf denjenigen Seiten, die dem zweiten Sensor 19 bzw. dem dritten Sensor 21 gegenüber liegen, sind die beiden Strahlteiler geschwärzt. Sowohl die Schwärzung als auch die Beschichtung mit einer Antireflexionsschicht haben den Zweck, Streulicht zu reduzieren oder zu vermeiden. Im Falle einer einfachen Ausbildung als Glasscheibe wären diese entlang der hier gezeigten Diagonalen - die Grenzflächen zwischen den beiden Prismen, welche die eigentliche strahlteilende Schicht bzw. den Strahlteiler bilden - angeordnet, d.h. im Winkel von 45° zur Einfallsrichtung des jeweiligen Lichtstrahls.

In der hier gezeigten Konfiguration sind der erste Strahlteiler 17 und der zweite Strahlteiler 20 im visuellen Spektralbereich teilweise reflektierend und im nahen Infrarot überwiegend transmittierend ausgebildet. Der erste Strahlteiler 17 ist hier bevorzugt für einen ersten Wellenlängenbereich zwischen 650 nm und 900 nm mindestens teilweise transparent. Der Tranmsissionsgrad im nahen Infrarot, umfassend den Wellenlängenbereich zwischen 750 nm und 900 nm liegt dabei bei mehr als 90 %, bevorzugt bei mehr als 95 %, wohingegen er für Wellenlängen zwischen 400 nm und 650 nm bei weniger als 10 %, bevorzugt weniger als 1 % und besonders bevorzugt bei weniger als 0,5 % liegt. Der zweite Strahlteiler 20 weist insbesondere im Zusammenhang mit der Erzeugung hyperspektraler Bilder für Wellenlängen zwischen 700 nm und 1100 nm idealerweise einen Transmissionsgrad von nahezu 100 % auf, wohingegen das Verhältnis von Transmission zu Reflexion im Wellenlängenbereich zwischen 400 nm und 600 nm zwischen 80:20 und 50:50 liegt und es bevorzugt nicht schwankt, d.h. konstant ist. Das Teilungsverhältnis ist abhängig vom Aufbau bzw. der Zusammensetzung der Beschichtung sowie der Wellenlänge.

Anhand der aus dem ersten Sensor 18 ausgelesenen Signale wird ein erstes monoskopisches Bild erzeugt und anhand der aus dem vierten Sensor 22 ausgelesenen Signale ein zweites monoskopisches Bild. Die Bildverarbeitungseinheit 23 kann dann die Daten des ersten und zweiten monoskopischen Bildes zur Verbesserung der Qualität des stereoskopischen Bildes verwenden, oder aber - alternativ oder in Ergänzung - ein drittes monoskopisches Bild erzeugen, was einem Betrachter dann zusätzlich angezeigt wird. Anstelle eines stereoskopischen Bildes ist es auch möglich, zwei monoskopische Bilder darzustellen, je nach Anforderungen des Operateurs. Diese sind bereits vorhanden, da sich das stereoskopische Bild aus einer Zusammensetzung zweier monoskopischer Bilder ergibt.

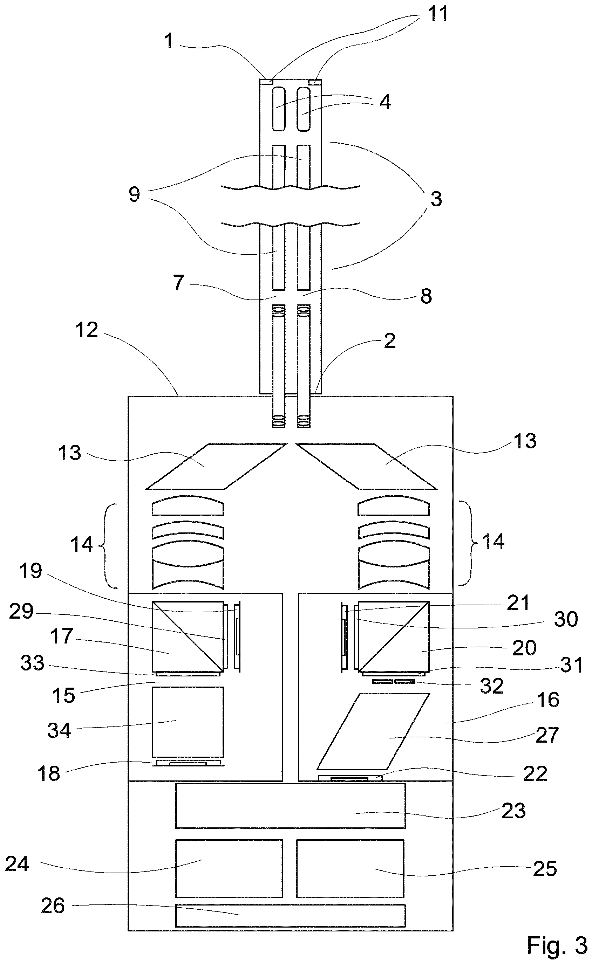

Fig. 3 zeigt eine Abwandlung des Stereoendoskops aus Fig. 2, bei dem die ersten Manipulationsmittel, der erste Sensor 18 sowie der erste Bildverarbeitungsalgorithmus so vorgegeben sind, dass ein Multiband-Bild erzeugt wird. Anstelle des ersten Filters 28 aus Fig. 2 wird hier ein Bandpassfilter 33 verwendet, welcher für mehrere enge Spektralbereiche durchlässig ist, in Kombination mit einer schmalbandigen Lichtquelle, die Licht in mehreren Spektralbereiche aussendet, für welche der Bandpassfilter 33 durchlässig ist. In einer Spektrometereinheit 34 wird das Licht spektral zerlegt entsprechend der Anzahl der Bänder. Der erste Sensor 18 kann in diesem Fall beispielsweise so angesteuert werden, dass benachbarte Pixel jeweils auf bestimmte, voneinander verschiedene Spektralbereiche, für die der Bandpassfilter 33 durchlässig ist, reagieren. Die Spektrometereinheit 34 kann auch so ausgestaltet sein, dass ausgangsseitig eine der Anzahl der Bänder entsprechende Anzahl von Bildern erzeugt wird, von denen ein jedes zu einem Band korrespondiert und in einen gesonderten Bereich des ersten Sensors 18 abgebildet wird.

Grundsätzlich ist es auch möglich, zwischen mindestens einem der beiden Strahlteiler und einem der diesem Strahlteiler zugeordneten Sensoren mindestens noch einen weiteren, hier nicht gezeigten, Strahlteiler anzuordnen, welcher Licht in einen weiteren Lichtstrahl auf einen weiteren Sensor ablenkt; im Zusammenhang damit sind dann auch weitere Manipulationsmittel zwischen dem weiteren Sensor und dem weiteren Strahlteiler angeordnet. Auf diese Weise lässt sich eine Kaskade von Strahlteilern und verschiedenen Manipulationsmethoden bzw. Analysemethoden realisieren.

Das vorangehend beschriebene Stereoendoskop lässt sich somit zu Erzeugung verbesserter stereoskopische Bilder von Gewebeproben verwenden. Wird beispielsweise ein erstes monoskopisches Bild erzeugt, indem Fluoreszenzsignale im infraroten Bereich detektiert werden, und ein zweites monoskopisches Bild erzeugt, in dem ein hyperspektrales Bild detektiert wird, lassen sich die beiden monoskopischen Bilder im Rahmen einer Bildverarbeitung beispielsweise zur Erhöhung des Kontrasts und/oder zur Verstärkung der Intensität bei dem stereoskopischen Bild verrechnen; zusätzlich lassen sich aufgrund der komplementären Analysemethoden auch weitere Informationen in das stereoskopische Bild aufnehmen. Ein Operateur an einem robotischen Operationssystem erhält auf diese Weise genauere Informationen über das beispielsweise zu entfernende Gewebe und kann seinen Eingriff genauer planen. Zudem wird beispielsweise bei der Tumorentfernung die Fehleranfälligkeit verringert, was das Entfernen von gesundem wie krankem Gewebe betrifft.

### Bezugszeichenliste

- 1: distales Ende
- 2: proximales Ende
- 3: Schaft
- 4: Eingangsobjektiv
- 5: optisches Abbildungssystem
- 6: erste Zwischenbildebene
- 7: erster Abbildungskanal
- 8: zweiter Abbildungskanal
- 9: optisches Übertragungssystem
- 10: zweite Zwischenbildebene
- 11: Beleuchtungsmittel
- 12: Analyseeinheit
- 13: Prisma
- 14: Adapteroptik
- 15: erste Sensoreinheit
- 16: zweite Sensoreinheit
- 17: erster Strahlteiler
- 18: erster Sensor
- 19: zweiter Sensor
- 20: zweiter Strahlteiler
- 21: dritter Sensor
- 22: vierter Sensor
- 23: Bildverarbeitungseinheit
- 24: erste Steuerungseinheit
- 25: zweite Steuerungseinheit
- 26: Anschlussinterface
- 27: hyperspektrales Spektrometer
- 28: erster Filter
- 29: zweiter Filter
- 30: dritter Filter
- 31: Gelbfilter
- 32: Spalt
- 33: Bandpassfilter
- 34: Spektrometereinheit
- 41: erster Lichtstrahl
- 42: zweiter Lichtstrahl
- 43: dritter Lichtstrahl
- 44: vierter Lichtstrahl

## Patentansprüche

1. Stereoendoskop zur Beobachtung und Analyse eines Objekts, umfassend
- einen Schaft (3) mit einem distalen Ende (1) und einem proximalen Ende (2)
- Beleuchtungsmittel, welche das Objekt vom distalen Ende (1) des Schaftes (3) her beleuchten,
- stereoskopische Abbildungsmittel, welche vom Objekt abgestrahltes Licht vom distalen Ende (1) zum proximalen Ende (2) übertragen,
- eine erste Sensoreinheit (15), auf welche Licht aus einem ersten Abbildungskanal (7) abgebildet wird, wobei die erste Sensoreinheit (15) einen ersten Strahlteiler (17) umfasst, welcher das Licht in einen ersten Lichtstrahl (41) und einen zweiten Lichtstrahl (42) aufteilt, sowie einen ersten Sensor (18) und einen zweiten Sensor (19), welche Licht des ersten Lichtstrahls (41) bzw. des zweiten Lichtstrahls (42) detektieren und in erste bzw. zweite elektromagnetische Signale umwandeln,
- und eine zweite Sensoreinheit (16), auf welche Licht aus einem zweiten Abbildungskanal (8) abgebildet wird, wobei die zweite Sensoreinheit (16) einen zweiten Strahlteiler (20) umfasst, welcher das Licht in einen dritten Lichtstrahl (43) und einen vierten Lichtstrahl (44) aufteilt, sowie einen dritten Sensor (21) und einen vierten Sensor (22), welche Licht des dritten Lichtstrahls (43) bzw. des vierten Lichtstrahls (44) detektieren und in dritte bzw. vierte elektromagnetische Signale umwandeln,
- wobei der zweite Sensor (19) und der dritte Sensor (21) gleichartig sind und jeweils Licht im sichtbaren Spektralbereich detektieren,
- eine Bildverarbeitungseinheit (23) zur Verarbeitung der ersten bis vierten elektromagnetischen Signale, welche die zweiten und dritten elektromagnetischen Signale zu einem stereoskopischen Bild verarbeitet, **dadurch gekennzeichnet, dass**
- in der ersten Sensoreinheit (15) zwischen dem ersten Strahlteiler (17) und dem ersten Sensor (18) erste Manipulationsmittel angeordnet sind, mit denen der erste Lichtstrahl (41) manipuliert wird,
- in der zweiten Sensoreinheit (16) zwischen dem zweiten Strahlteiler (20) und dem vierten Sensor (22) zweite, von den ersten verschiedene Manipulationsmittel angeordnet sind, mit denen der vierte Lichtstrahl (44) manipuliert wird,
- in der Bildverarbeitungseinheit (23) ein erster, auf die ersten Manipulationsmittel abgestimmter Bildverarbeitungsalgorithmus implementiert ist, welcher ein erstes monoskopisches Bild erzeugt, sowie ein zweiter, auf die zweiten Manipulationsmittel abgestimmter Bildverarbeitungsalgorithmus, welcher ein zweites monoskopisches Bild erzeugt,
- wobei erste und zweite Manipulationsmittel, erster Sensor (18) und vierter Sensor (22) sowie erster und zweiter Bildverarbeitungsalgorithmus so vorgegeben sind, dass das erste und zweite monoskopische Bild sowie das stereoskopische Bild komplementäre Bildinformationen enthalten.

2. Stereoendoskop nach Anspruch 1, **dadurch gekennzeichnet, dass** die Beleuchtungsmittel mehrere, mindestens eine erste und eine zweite Lichtquelle umfassen, von denen mindestens eine Lichtquelle monochromatisches, kohärentes Licht abstrahlend ausgebildet ist.

3. Stereoendoskop nach Anspruch 1 oder 2, **dadurch gekennzeichnet, dass** die ersten und zweiten Manipulationsmittel, der erste Sensor (18) und vierte Sensor (22) und der erste und zweite Bildverarbeitungsalgorithmus alternativ ausgebildet sind zur Erzeugung
i. eines Fluoreszenzbildes aus Fluoreszenzsignalen,
ii. eines hyperspektralen Bildes,
iii. eines polarisationsaufgelösten Bildes,
iv. eines Raman-spektroskopischen Bildes,
v. eines optisch-kohärenztomographischen Bildes,
vi. eines Speckle-Kontrast-Bildes,
vii. eines Multiband-Bildes, oder
viii. eines Narrowband-Bildes.

4. Stereoendoskop nach einem der Ansprüche 1 bis 3, **dadurch gekennzeichnet, dass** der erste Strahlteiler (15) und der zweite Strahlteiler (16) unterschiedlich ausgebildet sind.

5. Stereoendoskop nach Anspruch 1 bis 4, bei dem die ersten Manipulationsmittel, der erste Sensor (18) und der erste Bildverarbeitungsalgorithmus zur Erzeugung eines Fluoreszenzbildes aus Fluoreszenzsignalen im infraroten Bereich ausgebildet sind, **dadurch gekennzeichnet, dass** der erste Sensor (18) für die Detektion des Fluoreszenzspektrums von ICG (Indocyaningrün) ausgelegt ist.

6. Stereoendoskop nach einem der Ansprüche 1 bis 5, bei dem die zweiten Manipulationsmittel, der vierte Sensor (22) und der zweite Bildverarbeitungsalgorithmus zur Erzeugung eines hyperspektralen Bildes ausgebildet sind und die zweiten Manipulationsmittel ein hyperspektrales Spektrometer (27) umfassen.

7. Stereoendoskop nach einem der Ansprüche 1 bis 6, **dadurch gekennzeichnet, dass** der erste Strahlteiler (17) und der zweite Strahlteiler (20) entweder im visuellen Spektralbereich teilweise reflektierend und im nahen Infrarotbereich überwiegend transmittierend, oder im visuellen Spektralbereich überwiegend transmittierend und im nahen Infrarotbereich teilweise reflektierend, ausgebildet sind.

8. Stereoendoskop nach Anspruch 7, **dadurch gekennzeichnet, dass** der erste Strahlteiler (17) für einen ersten Wellenlängenbereich zwischen 650 nm und 900 nm mindestens teilweise transparent ist, und der Transmissionsgrad für Wellenlängen zwischen 400 nm und 650 nm weniger als 10 % beträgt.

9. Stereoendoskop nach Anspruch 7 oder 8, **dadurch gekennzeichnet, dass** der Transmissionsgrad des ersten Strahlteilers (17) für Wellenlängen zwischen 400 nm und 650 nm weniger als 0,5 % und für Wellenlängen zwischen 750 nm und 900 nm mehr als 95 % beträgt.

10. Stereoendoskop nach Anspruch 7, **dadurch gekennzeichnet, dass** der zweite Strahlteiler (20) für Wellenlängen zwischen 700 nm und 1100 nm einen Transmissionsgrad von mehr als 90 %, bevorzugt nahezu 100 % aufweist.

11. Stereoendoskop nach Anspruch 10, **dadurch gekennzeichnet, dass** bei dem zweiten Strahlteiler (20) das Verhältnis von Transmission zu Reflexion im Wellenlängenbereich zwischen 400 nm und 600 nm konstant zwischen 80:20 und 50:50 liegt.

12. Stereoendoskop nach einem der Ansprüche 1 bis 11, **dadurch gekennzeichnet, dass** zwischen mindestens einem der beiden Strahlteiler (17, 20) und einem der diesem Strahlteiler zugeordneten Sensoren mindestens ein weiterer Strahlteiler angeordnet ist, der Licht in einen weiteren Lichtstrahl auf einen weiteren Sensor ablenkt, sowie weitere Manipulationsmittel zwischen dem weiteren Sensor und dem weiteren Strahlteiler angeordnet sind.

13. Stereoendoskop nach einem der Ansprüche 1 bis 12, **dadurch gekennzeichnet, dass** die Bildverarbeitungseinheit (23) dazu ausgelegt ist, Daten des ersten und des zweiten monoskopischen Bildes zur Verbesserung der Qualität des stereoskopischen Bildes zu verwenden oder ein drittes monoskopisches Bild zu erzeugen.

14. Stereoendoskop nach einem der Ansprüche 1 bis 13, **dadurch gekennzeichnet, dass** die erste Sensoreinheit (15) und die zweite Sensoreinheit (16) als Steckmodule ausgebildet sind.

15. Verwendung eines Stereoendoskops nach einem der Ansprüche 1 bis 14 zur Erzeugung verbesserter stereoskopischer Bilder von Gewebeproben, bei dem ein erstes monoskopisches Bild erzeugt wird, indem Fluoreszenzsignale detektiert werden, und ein zweites monoskopisches Bild erzeugt wird, indem ein hyperspektrales Bild detektiert wird, und erstes und zweites monoskopisches Bild bei einer Bildverarbeitung zur Erhöhung des Kontrasts und/oder zur Verstärkung der Intensität und/oder zur verbesserten Unterscheidung von Gewebearten bei dem stereoskopischen Bild verrechnet werden.
